# EUROPEAN PATENT APPLICATION

(11) **EP 3 884 982 A1**
(43) Date of publication of application: **29.09.2021**
(21) Application number: 20315046.1
(22) Date of filing: 25.03.2020
(51) Int. Cl.: A61M 16/00, A61M 16/20

(54) **MANUAL ARTIFICIAL RESPIRATION BAG WITH FLOW-RESISTANCE SETTING MEANS COOPERATING WITH A ONE-WAY VALVE**

(71) Applicant: Air Liquide Medical Systems, 92160 Antony (FR)
(72) Inventor: Alberici, Luca, 25073 Bovezzo (IT); Richard, Jean-Christophe, 92160 Antony (FR); Zadra, Davide, 25073 Bovezzo (IT); Badat, Bilal, 92160 Antony (FR); Massaro, Paolo, 25073 Bovezzo (IT); Lesimple, Arnaud, 92160 Antony (FR)
(74) Representative: Air Liquide

(57) **Abstract**

The invention concerns a manual artificial respiration bag (1) comprising a deformable bag (2) comprising a gas inlet (4), a gas outlet (3) and an inner volume (5) for a respiratory gas, a downstream conduct element (100) fluidly connected to the gas outlet (3) of the deformable bag (2), and comprising an exhaust valve (110), and a downstream one-way valve (50, 55) arranged into the downstream conduct element (100), said downstream one-way valve (50, 55) being configured for allowing a flow of respiratory gas to pass through said downstream one-way valve (50, 55) only toward the exhaust valve (110). It further comprises flow-resistance setting means (120) cooperating with the downstream one-way valve (50, 55) for setting or adjusting a desired flow resistance level of said downstream one-way valve (50, 55) thereby controlling the flow of respiratory gas passing through said downstream one-way valve (50, 55).

## Description

The present invention relates to a multifunctional manual artificial respiration bag, such as manual resuscitation devices/systems, resuscitators or the like, that can be used for providing a respiratory gas to a person, such as a patient.

A manual resuscitation bag can be used for providing a respiratory gas, such as air, oxygen or a mixture thereof, to a person in need of a respiratory assistance, typically a patient. However, current manual artificial respiration bags are not useable for delivering gas to patients in various situations as they are generally dedicated for a given or specific task.

Thus, the most classical manual artificial respiration bags can only be used for providing a respiratory gas, such as air or an air/O₂ mixture, to a patient, during his/her transportation or transfer from a first place, such as an operation room or a radiotherapy room, to a second place, such as a recovery room or a bedroom, or vice versa. Examples of such bags are given by US-A-3,063,620, US-A-2017/0157348, US-A-4,501,271 or US-A-2,834,339.

However, such classical manual artificial respiration bags are not suitable for providing a respiratory gas to a person in state of cardiac arrest as they are not compatible or well-adapted with the cardiac massage that is done on the person.

This is why, some manual artificial respiration bags, called manual resuscitation bags, have been specifically designed for artificially ventilating a person in state of cardiac arrest, while thoracic compressions (TC), i.e. successive compressions and decompressions, are exerted by a rescuer on the thoracic cage of said person for restoring gas exchanges in the lungs and a blood circulation in the body and toward the organs, especially to the brain of the patient.

Examples of such manual resuscitation bags dedicated to "cardiac arrest" are given by WO-A-2019/001751 and WO-A-2019/001752. With such a manual resuscitation bag, it is possible to provide air or air/O₂ mixtures, even during chest compressions/decompressions. Other examples of manual resuscitation bags are described in WO-A-2017/096286, WO2015/041396, WO2005/035065 and EP-A-0743075.

A goal of the present invention is to provide an improved manual artificial respiration bag, namely a "multiuse" or "multifunctional" manual artificial respiration bag, that can be used in various situations for specific tasks including transportation/transfer of patients from a first place to a second place, in an hospital, in the field or others locations, as well as with persons in state of cardiac arrest that undergo thoracic compressions.

A solution according to the present invention concerns a manual artificial respiration bag comprising:
- a deformable bag, i.e. flexible bag, comprising a gas inlet, a gas outlet and an inner volume for a respiratory gas,
- a downstream conduct element fluidly connected to the gas outlet of the deformable bag, and comprising an exhaust valve, and
- a downstream one-way valve arranged into the downstream conduct element, said downstream one-way valve being configured for allowing a flow of respiratory gas to pass through said downstream one-way valve only toward the exhaust valve,
characterized in that it further comprises flow-resistance setting means cooperating with the downstream one-way valve for setting or adjusting a desired flow resistance level of said downstream one-way valve thereby controlling the flow of respiratory gas passing through said downstream one-way valve.

Depending on the embodiment, a manual artificial respiration bag according to the present invention can comprise of one or several of the following additional features:
- the downstream conduct element comprises a inner passage or lumen.
- the downstream conduct element may optionally comprise an over-pressure valve.
- the downstream one-way valve is arranged into the downstream conduct element (i.e. in its lumen), for instance between the over-pressure valve (when present) and the exhaust valve.
- the flow-resistance setting means comprise a flow-resistance setting device.
- the flow-resistance setting means comprise a mobile adjusting member, actuatable by a user, comprising a bearing surface acting on the downstream one-way valve for setting or adjusting the flow resistance level of said downstream one-way valve.
- the bearing surface of the adjusting member has an annular shape, preferably a circular shape.
- the mobile adjusting member is rotatable, i.e. configured for being mobile in rotation, when turned clockwise or counterclockwise by a user.
- the downstream one-way valve comprises a valve-support arranged into the downstream conduct element, i.e. in its lumen, and a flexible valve body.
- the valve-support is rigid.
- the mobile adjusting member acts on (i.e. is configured for acting on) the flexible valve body when actuated by the user.
- the flexible valve body is sandwiched between the mobile adjusting member and the valve support.
- the flexible valve body of the downstream one-way valve has an umbrella-shape.
- the flexible valve body of the downstream one-way valve is made of a flexible material, such as an elastomer.
- the flexible valve body has an umbrella-shape comprising a disk-shape body and a rod element integral with said disk-shape body, preferably fixed at the center of said disk-shape body and projecting away from said disk-shape body.
- the valve-support comprises a support orifice.
- the rod element of the flexible valve body traverses the support orifice of the valve-support, i.e. the rod element is lodged into said support orifice
- the bearing surface of the mobile adjusting member, when actuated by the user, acts on the disk-shape body of the flexible valve body of the downstream one-way valve, thereby setting or adjusting the flow resistance level of said downstream one-way valve.
- the bearing surface of the mobile adjusting member, when actuated by the user, pushes against the disk-shape body of the flexible valve body thereby slightly deforming, i.e. bending, said disk-shape body.
- the adjusting member comprises at least one guiding-pin and the downstream conduct element comprises at least one guiding-groove, said guiding-pin being guided by and in said guiding-groove when the adjusting member is moved/actuated, typically rotated, by the user, preferably (at least) two guiding-pins and (at least) two guiding-groove are provided.
- it further comprises a mobile closing flap.
- the exhaust valve comprises an exhaust port.
- said closing flap cooperates with said exhaust port for at least partially closing said exhaust port, i.e. the exhaust port is more or less open depending on the position of the mobile closing flap.
- the closing flap is preferably arranged on (i.e. carried by) the adjusting member so that the closing flap at least partially closes said exhaust port in response to an actuation of the adjusting member by the user, typically to a rotation of the adjusting member by the user.
- it further comprises an upstream conduct element fluidly connected to the gas inlet of the deformable bag.
- the upstream conduct element comprises a PEP exhaust valve fluidly communicating with the ambient atmosphere for venting gas to the atmosphere when the gas pressure, into the upstream conduct element, exceeds a given pressure threshold.
- the upstream conduct element further comprises an air admission valve in fluid communication with the ambient atmosphere for allowing air to enter into the upstream conduct element.
- the upstream conduct element further comprises an oxygen port for fluidly connecting an oxygen source for providing oxygen.
- it further comprises an oxygen source fluidly connected to the oxygen port of the upstream conduct element, such as an oxygen-containing cylinder.
- the PEP exhaust valve arranged in the upstream conduct element comprises PEP-setting means for setting the desired pressure threshold.
- the upstream conduct element further comprises a reservoir port for fluidly connecting a flexible gas reservoir.
- it further comprises a flexible gas reservoir fluidly connected to the reservoir port of the upstream conduct element.
- the first conduct element comprises an oxygen port or entry arranged between the reservoir port for connecting the gas reservoir and the upstream one-way valve.
- the downstream conduct element further comprises an interface port for fluidly connecting a respiratory interface.
- it further comprises a respiratory interface fluidly connected the interface port of the downstream conduct element, preferably by means of a ball-head connector or the like.
- it further comprises an upstream one-way valve arranged into the upstream conduct element between the deformable bag and the PEP exhaust valve, said upstream one-way valve being configured for allowing a flow of respiratory gas to pass through said upstream one-way valve only toward the deformable bag.
- it further comprises a flow-restriction element arranged into the downstream conduct element between the deformable bag and the over-pressure valve.
- the PEP exhaust valve arranged in the upstream conduct element comprises PEP-setting means for setting the desired pressure threshold.
- the PEP exhaust valve comprises a valve body and means for setting a desired pressure threshold comprising a rotatable member, actuatable by the user, arranged on the valve body and cooperating with pressure adjusting means arranged into the valve body.
- the opening pressure of PEP exhaust valve is of between 0 cm H₂O and 30 cm H₂O, preferably of between 0 cm H₂O and 15 cm H₂O.
- the PEP exhaust valve comprises markings corresponding to several settable pressure values, in particular several settable pressure values of between 0 cm H₂O and 30 cm H₂O, for instance several pressure values comprising 0, 5 and 10 cmH₂O.
- the rotatable member of the PEP exhaust valve comprises an inner axially-projecting bulb cooperating with the pressure adjusting means arranged into the valve body.
- the pressure adjusting means arranged into the valve body comprise a piston head, a spring element and a valve seat cooperating with the piston head for adjusting the pressure threshold.
- the upstream conduct element comprises a inner passage or lumen.
- the valve body of the PEP exhaust valve is in fluid communication with the lumen of the first upstream conduct element.
- it further comprises a gas delivery conduct comprising the interface port and in fluid communication with the downstream conduct element for conveying at least part of the gas circulating into the gas conduct to a patient interface.
- the patient interface comprises of a respiratory mask or a tracheal cannula or probe.
- the overpressure valve arranged in the downstream conduct element is configured to vent to the atmosphere at least part of the gas present in the gas conduct, when the gas pressure in the downstream conduct element exceeds a given value.

Some embodiments according to the present invention are shown in the enclosed Figures, among which:
- Figures 1 and 2 represent side views of an embodiment of a manual artificial respiration bag according to the present invention,
- Figure 3 is an exploded scheme of a manual artificial respiration bag of according to the present invention,
- Figure 4 is an enlarged partial view of Figure 3,
- Figure 5 is an enlarged partial view of Figure 4,
- Figures 6 and 7 show the rotatable member of the resistance setting means in a first position, wherein the exhaust port is fully open (i.e. not closed),
- Figures 8 and 9 show the rotatable member of the resistance setting means in a second position, wherein the exhaust port is partially closed,
- Figures 10 and 11 are schemes of the downstream conduct element and the resistance setting means of a manual artificial respiration bag of according to the present invention, shown in the first and second positions, respectively,
- Figure 12 is a cross-sectional view of the downstream conduct element and the resistance setting means of a manual artificial respiration bag of according to the present invention,

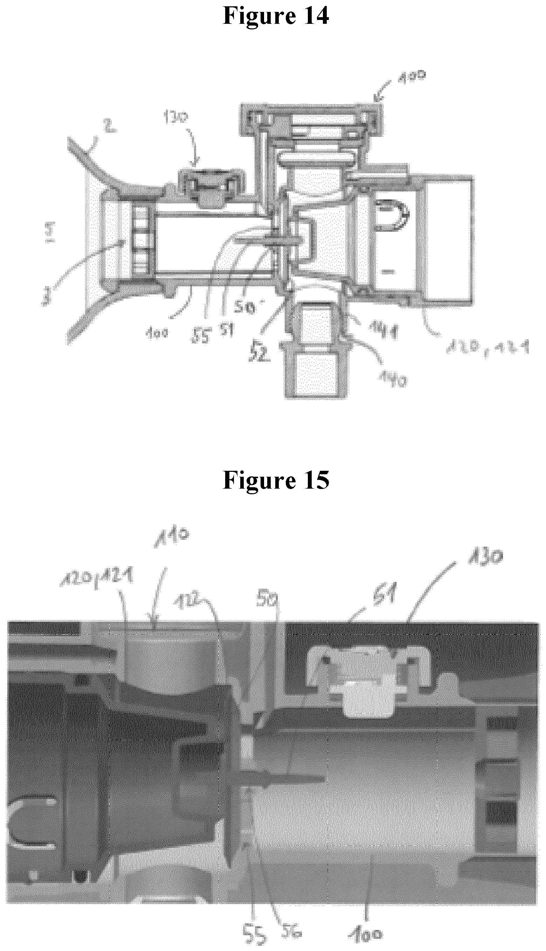

- Figures 13 and 14 are other cross-sectional views of the downstream conduct element of a manual artificial respiration bag of according to the present invention, showing the cooperation between flow-resistance setting means and downstream one-way valve, and

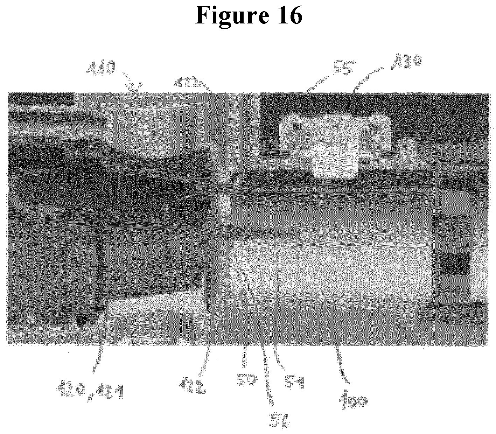

- Figures 15 and 16 are enlarged cross-sectional (partial) views of the downstream conduct element of a manual artificial respiration bag of according to the present invention, showing the cooperation between flow-resistance setting means and downstream one-way valve.

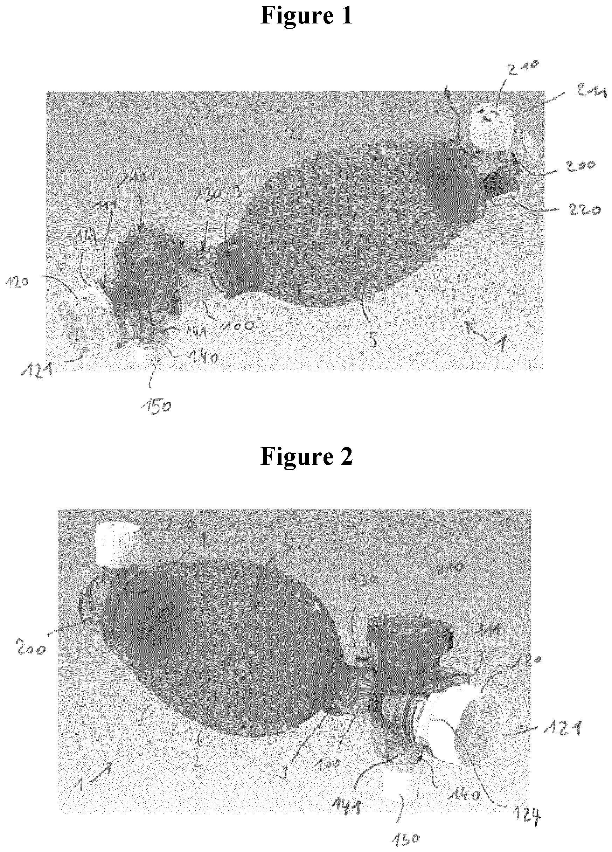

Figures 1 and 2 represent side views of an embodiment of a manual artificial respiration bag 1 according to the present invention.

Said manual artificial respiration bag 1 generally comprises a deformable bag 2, i.e. flexible hollow bag, comprising an inner volume 5 for receiving a respiratory gas, such as air or a mixture of air and oxygen, on which a user, such as a rescuer (e.g. a physician), can exert a manual-pressure, i.e. that can be manually squeeze, for providing the respiratory gas to a patient, i.e. expelling the gas contained into the deformable bag 2 toward a patient in need thereof.

The deformable bag 2 further comprises a gas inlet 4 for introducing a respiratory gas into the inner volume 5 of the deformable bag 2 and a gas outlet 3 for delivering gas, while the deformable bag 2 is squeezed by a user. The deformable bag 2 is typically made of flexible material, typically a polymer material and has an inner volume 5 of preferably less than 2 L (i.e. when filled with water), for instance of about 1 L.

As shown in Figures 1 and 2, the manual artificial respiration bag 1 further comprises an upstream conduct element 200 fluidly connected to the gas inlet 4 of the deformable bag 2, and a downstream conduct element 100 fluidly connected to the gas outlet 3 of the deformable bag 2.

Both upstream and downstream conduct elements 200, 100 have a generally-tubular shape comprising a lumen for conveying the gas.

The upstream conduct element 200 comprises a PEP exhaust valve 210 fluidly communicating with the ambient atmosphere for venting gas (i.e. an over-pressure) to the atmosphere when the gas pressure, into the upstream conduct element 200, i.e. in its lumen, exceeds a given pressure threshold. In other words, the PEP exhaust valve 210 prevents gas overpressures in the deformable bag 2 and/or in the upstream conduct element 200 fluidly connected to the gas inlet 4 of the deformable bag 2.

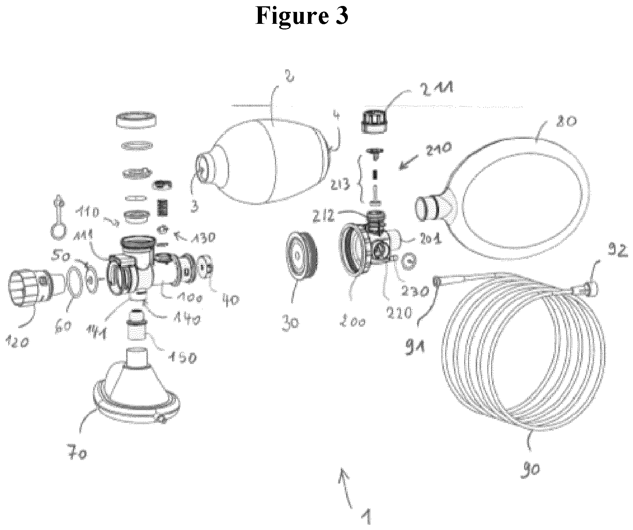

The upstream conduct element 200 further comprises an air admission valve 220 in fluid communication with the ambient atmosphere, for providing ambient air to the upstream conduct element 200, and preferably an oxygen port 230 for connecting an oxygen source thereto, such as an oxygen cylinder, for providing additional oxygen to the upstream conduct element 200 and thereby obtaining an oxygen/air mixture. The oxygen source is fluidly connected to the oxygen port 230 by means of a gas line 90 comprising gas connectors 91 or plugs, such as a flexible hose or the like, as shown in Figure 3.

Further, an upstream one-way valve 30 is arranged into the upstream conduct element 200 between the deformable bag 2 and the PEP exhaust valve 210, which is configured for allowing a flow of respiratory gas to pass through said upstream one-way valve 30 only toward the deformable bag 2, i.e. in the direction of the deformable bag 2.

The upstream conduct element 200 also comprises a reservoir port 201 for fluidly connecting a flexible gas reservoir 80, as shown in Figure 3.

Furthermore, the downstream conduct elements 100 comprises an exhaust valve 110 with an exhaust port 111 for venting to the atmosphere, the CO₂-enriched gases expired by the patient and/or coming out of the lungs of the patient.

Optionally, in some embodiments, it may also comprise an over-pressure valve 130 for venting to the atmosphere any over pressure in said downstream conduct elements 100, i.e. in its lumen.

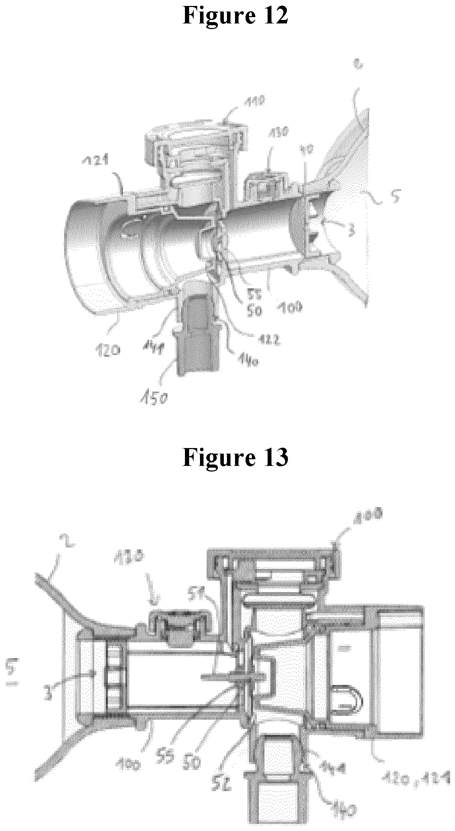

As shown in Figures 3-5 and 12, a downstream one-way valve 50, 55 is arranged into the downstream conduct element 100, i.e. into its lumen, for instance between the over-pressure valve 130 (when present) and the exhaust valve 110, and is configured for allowing a flow of respiratory gas to pass through said downstream one-way valve 50, 55 only toward the exhaust valve 110. In other words, the role of the downstream one-way valve 50, 55 is to control the way that the gas circulates into the downstream one-way valve 50, 55.

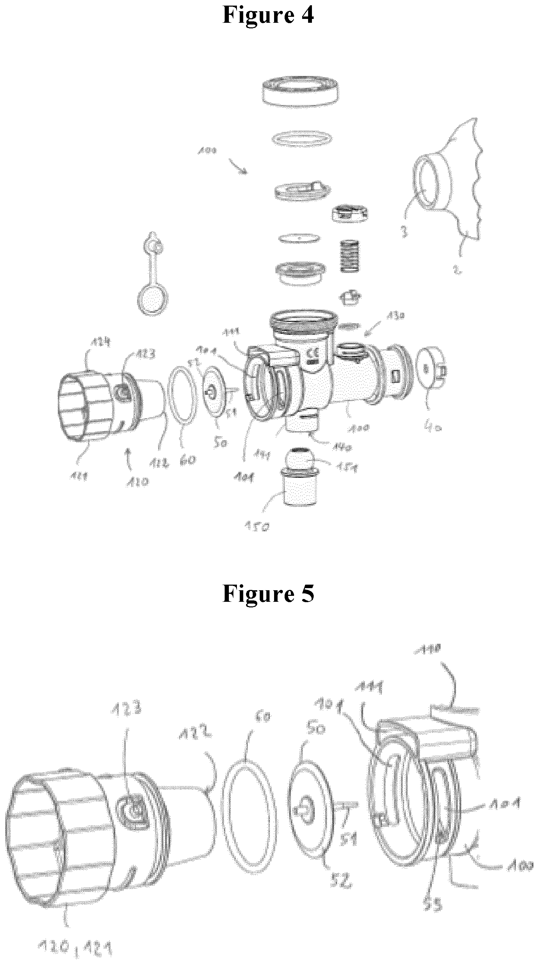

The respiratory gas, such as air or an air/O₂ mixture, flowing out of the deformable bag 2, when squeezed by a medical staff for instance, passes through the downstream conduct element 100 that is fluidly connected to the gas outlet 3 of the deformable bag 2, and is subsequently delivered to the patient's airways, by means of a respiratory interface 70, such as a facial mask, a laryngeal mask, an endotracheal tube or the like that fluidly connected to an interface port 140 of the downstream conduct element 100, as illustrated in Figures 3 and 4, preferably by means of a ball-head 151 hollow connector 150 or any other suitable tubular-connector. The interface port 140 of the downstream conduct element 100 can be arranged on a gas delivery conduct 141 branched to the downstream conduct element 100.

Advantageously, the manual resuscitation bag 1 can comprise a handle (not shown) or the like for transporting it.

The PEP exhaust valve 210 comprises a rotatable member 211, such as a rotating knob or the like, actuatable by a user, namely a rescuer, a valve body 212 and means 213 for setting a desired pressure threshold including pressure adjusting means arranged into the valve body 212. Said pressure adjusting means 213 comprise a piston head, a spring element, such as a cylindrical spring, and a valve seat cooperating with the piston head for adjusting the pressure threshold as shown in Figure 3. The PEP exhaust valve 210 further comprises several markings corresponding to several settable pressure values, typically overpressure values of between 0 and 30 cm H₂O. In this aim, the rotatable member 211 further comprises an inner axially-projecting bulb (not visible), which cooperates with the pressure adjusting means 213 for adjusting the pressure threshold.

Further, the manual resuscitation bag 1 according to the invention can also comprise additional elements or features as explained below.

Thus, as shown in Figures 1-3, the upstream conduct element 200 further comprises an air admission valve 220 in fluid communication with the ambient atmosphere, an oxygen port 230 or entry for fluidly connecting a source of an oxygen-containing gas, such as or including a gas cylinder containing oxygen, which is delivered during insufflation phases. Such source of an oxygen-containing gas can be fluidly connected, via an oxygen line, such as a gas conduct, to the oxygen port 230 of the upstream conduct element 200. In this case, the flexible bag 2 can be filled with a mixture of oxygen and ambient air provided by the air admission valve 220 in fluid communication with the ambient atmosphere.

Furthermore, the downstream conduct element 100 fluidly connected to the gas outlet 3 of the deformable bag 2, comprises an exhaust valve 110 and a downstream one-way valve 50, 55 configured for allowing a flow of respiratory gas to pass through said downstream one-way valve 50, 55 only toward the exhaust valve 110, while circulating into the lumen of said downstream conduct element 100.

According to the present invention, it is also provided flow-resistance setting means 120 cooperating with the downstream one-way valve 50, 55 for setting or adjusting a desired flow resistance level of said downstream one-way valve 50, 55 thereby controlling the flow of respiratory gas passing through said downstream one-way valve 50, 55.

The flow-resistance setting means 120 comprise a mobile adjusting member 121, actuatable by a user, comprising a bearing surface 122 acting on the downstream one-way valve 50, 55, for setting or adjusting the flow resistance level of the downstream one-way valve 50, 55. Preferably, the mobile adjusting member 121 is rotatable, i.e. can be turned clockwise or counter-clockwise by the user.

As better shown in Figures 4 and 5, the downstream one-way valve 50, 55 comprises a valve support 55 arranged into the downstream conduct element 100 and a flexible valve body 50. The mobile adjusting member 121 acts directly or indirectly on the flexible valve body 50, when actuated by the user. The flexible valve body 50 is sandwiched between the mobile adjusting member 121 and the valve support 55.

In the embodiment shown, the flexible valve body 50 of the downstream one-way valve 50, 55 has an umbrella-shape comprising a disk-shape body 52 and a rod element 51 integral with said disk-shape body 52, whereas the valve-support 55 comprises a support orifice 56, the rod element 51 of the flexible valve body 50 traversing said support orifice 56 of the valve-support 55, i.e. the rod element 51 is positioned into the support orifice 56.

The mobile adjusting member 121 comprises a bearing surface 122, i.e. front surface, that acts more or less on the disk-shape body 52 of the flexible valve body 50 of the downstream one-way valve 50, 55, thereby setting or adjusting the flow resistance level of said downstream one-way valve 50, 55, depending on the force applied by the bearing surface 122 on the disk-shape body 52 of the downstream one-way valve 50, 55. In other words, when the user turns, i.e. provide a clockwise or counter-clockwise motion, the bearing surface 122 translates toward or backward with respect to the disk-shape body 52 of the flexible valve body 50.

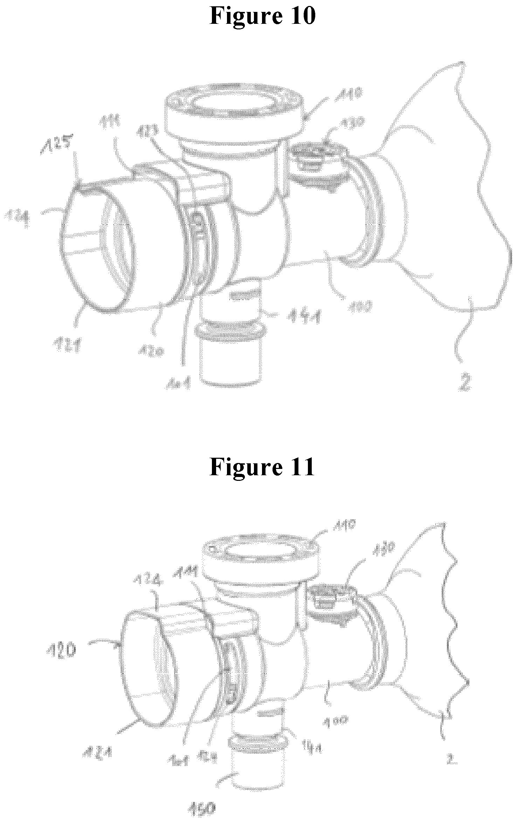

Preferably, as visible in Figures 4, 5, 10 and 11, the adjusting member 121 further comprises one or several guiding-pins 123, whereas the downstream conduct element 100 comprises one or several guiding-grooves 101 that receive and guide the guiding-pins 123 and hence the motion of the adjusting member 121, when the adjusting member 121 is rotated by the user, for instance two guiding-pins 123 cooperating with two guiding-groove 101.

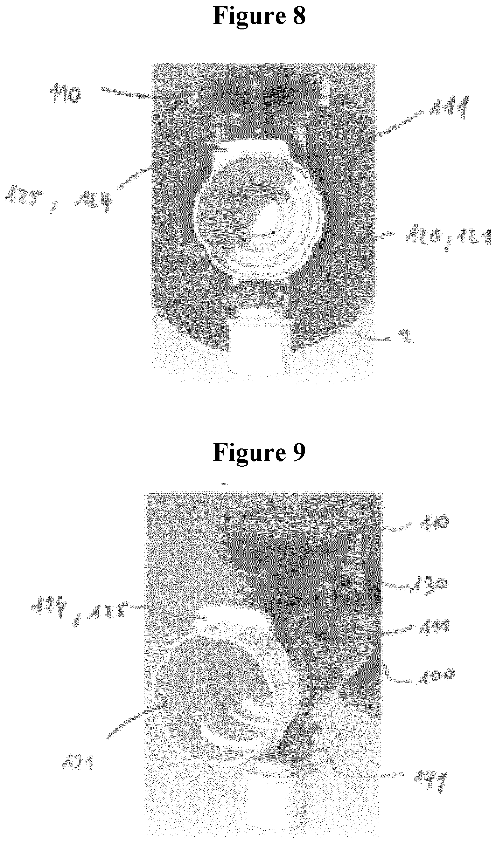

Furthermore, in Figures 4-11 the manual resuscitation bag 1 according to the invention also preferably comprises mobile port-closing means 124, actuatable by the user, cooperating with the exhaust port 111 of the exhaust valve 110 for at least partially closing said exhaust port 111, thereby controlling the flow of respiratory gas passing through the exhaust port 111 of the exhaust valve 110.

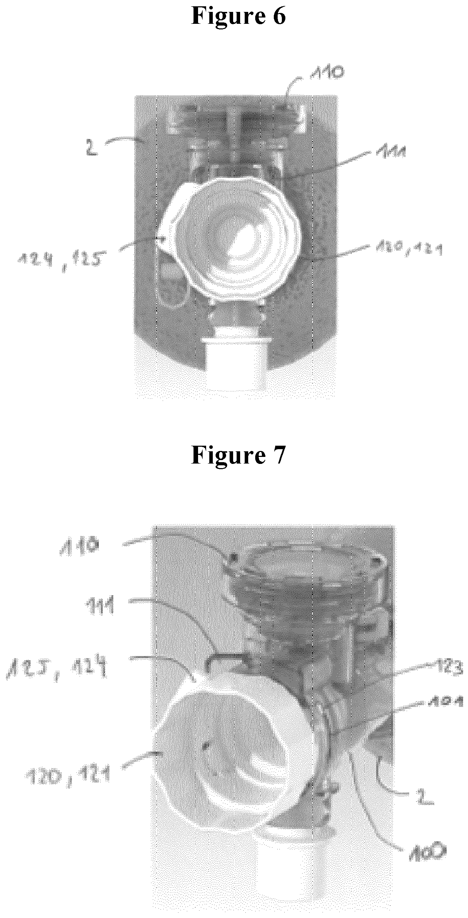

Preferably, said mobile port-closing means 124 are integrally-arranged on the rotatable adjusting member 121 actuatable by the user as shown in Figure 7-9. In an embodiment, the mobile port-closing means 124 comprise a closing flap or wall 125.

The closing flap 125 can be fixed to or carried by the outer wall 126 of the rotatable adjusting member 121. For instance, said closing flap or wall 125 can be made in one-piece, for instance molded in one-piece, with the outer wall 126 of the rotatable adjusting member 121. In other words, the outer wall 126 of the rotatable adjusting member 121 can be configured or designed to exhibit such a closing flap or wall 125.

According to another embodiment, said mobile port-closing means 124 can be fixed to another part of the manual resuscitation bag 1, in particular of the downstream conduct 100, i.e. not associated to or integral with the rotatable adjusting member 121.

Generally speaking, said mobile port-closing means 124 cooperate with the exhaust port 111 of the exhaust valve 110 for either not closing it, or partially closing said exhaust port 111, thereby limiting (or not) the flow of respiratory gas passing through said exhaust port 111 of the exhaust valve 110, in particular during the expiration phases of a patient.

When arranged on the adjusting member 121, the closing flap 125 moves and at least partially closes the exhaust port 111 in response to an actuation of the adjusting member 121 by the user, typically to a rotation of the adjusting member 121 by the user.

Furthermore, as shown in Figures 3 and 4, it is also provided a flow-restriction element 40, such as a disk element carrying a calibrated orifice that is arranged into the downstream conduct element 100 downstream of the deformable bag 2, for regulating the flow of gas delivered by the flexible reservoir 2.

The manual artificial respiration bag of the present can be used in various situations, for instance for resuscitating a person in state of cardiac arrest or the like, or for ventilation a person during transportation from one place to another place, in the field, in hospital, at the patient's home, in emergency vehicles or in any other place.

## Claims

1. Manual artificial respiration bag (1) comprising:
- a deformable bag (2) comprising a gas inlet (4), a gas outlet (3) and an inner volume (5) for a respiratory gas,
- a downstream conduct element (100) fluidly connected to the gas outlet (3) of the deformable bag (2), and comprising an exhaust valve (110), and
- a downstream one-way valve (50, 55) arranged into the downstream conduct element (100), said downstream one-way valve (50, 55) being configured for allowing a flow of respiratory gas to pass through said downstream one-way valve (50, 55) only toward the exhaust valve (110),
**characterized in that** it further comprises flow-resistance setting means (120) cooperating with the downstream one-way valve (50, 55) for setting or adjusting a desired flow resistance level of said downstream one-way valve (50, 55) thereby controlling the flow of respiratory gas passing through said downstream one-way valve (50, 55).

2. Manual artificial respiration bag according to the preceding claim, **characterized in that** the flow-resistance setting means (120) comprise a mobile adjusting member (121), actuatable by a user, comprising a bearing surface (122) acting on the downstream one-way valve (50, 55) for setting or adjusting the flow resistance level of said downstream one-way valve (50, 55).

3. Manual artificial respiration bag according to any one of the preceding claims, **characterized in that** the mobile adjusting member (121) is rotatable.

4. Manual artificial respiration bag according to any one of the preceding claims, **characterized in that** the downstream one-way valve (50, 55) comprises a valve support (55) arranged into the downstream conduct element (100) and a flexible valve body (50), the mobile adjusting member (121) acting on the flexible valve body (50), preferably the flexible valve body (50) is sandwiched between the mobile adjusting member (121) and the valve support (55).

5. Manual artificial respiration bag according to any one of the preceding claims, **characterized in that** the flexible valve body (50) of the downstream one-way valve (50, 55) has an umbrella-shape.

6. Manual artificial respiration bag according to any one of the preceding claims, **characterized in that**:
- the flexible valve body (50) of the downstream one-way valve (50, 55) has an umbrella-shape comprising a disk-shape body (52) and a rod element (51) integral with said disk-shape body (52), and
- the valve-support (55) comprises a support orifice (56), the rod element (51) of the flexible valve body (50) traversing said support orifice (56) of the valve-support (55).

7. Manual artificial respiration bag according to any one of the preceding claims, **characterized in that** the bearing surface (122) of the mobile adjusting member (121) acts on the disk-shape body (52) of the flexible valve body (50) of the downstream one-way valve (50, 55), when actuated by the user, thereby setting or adjusting the flow resistance level of said downstream one-way valve (50, 55).

8. Manual artificial respiration bag according to any one of the preceding claims, **characterized in that** the adjusting member (121) comprises at least one guiding-pin (123) and the downstream conduct element (100) comprises at least one guiding-groove (101), said guiding-pin (123) being guided by and in said guiding-groove (101) when the adjusting member (121) is rotated by the user, preferably two guiding-pins (123) and two guiding-groove (101).

9. Manual artificial respiration bag according to any one of the preceding claims, **characterized in that** it further comprises mobile port-closing means (124), actuatable by the user, cooperating with the exhaust port (111) of the exhaust valve (110) for at least partially closing said exhaust port (111), thereby controlling the flow of respiratory gas passing through the exhaust port (111) of the exhaust valve (110).

10. Manual artificial respiration bag according to any one of the preceding claims, **characterized in that** the mobile port-closing means (124) are arranged on the adjusting member (121) so that said mobile port-closing means (124) at least partially close said exhaust port (111) in response to an actuation of the adjusting member (121) by the user, typically to a rotation of the adjusting member (121) by the user.

11. Manual artificial respiration bag according to any one of the preceding claims, **characterized in that** it further comprises an upstream conduct element (200) fluidly connected to the gas inlet (4) of the deformable bag (2), said upstream conduct element (200) comprising :
- a PEP exhaust valve (210) fluidly communicating with the ambient atmosphere for venting gas to the atmosphere when the gas pressure, into the upstream conduct element (200), exceeds a given pressure threshold, and
- an air admission valve (220) in fluid communication with the ambient atmosphere,
- and/or an oxygen port (230) for connecting an oxygen source.

12. Manual artificial respiration bag according to claim 11, **characterized in that** the PEP exhaust valve (210) arranged in the upstream conduct element (200) comprises PEP-setting means for setting the desired pressure threshold.

13. Manual artificial respiration bag according to any one of the preceding claims, **characterized in that** :
- the upstream conduct element (200) further comprises a reservoir port (201) for fluidly connecting a flexible gas reservoir (80), and
- the downstream conduct element (100) further comprises an interface port (140) for fluidly connecting a respiratory interface (70).

14. Manual artificial respiration bag according to any one of the preceding claims, **characterized in that** it further comprises :
- an upstream one-way valve (30) arranged into the upstream conduct element (200) between the deformable bag (2) and the PEP exhaust valve (210), said upstream one-way valve (30) being configured for allowing a flow of respiratory gas to pass through said upstream one-way valve (30) only toward the deformable bag (2), and/or
- a flow-restriction element (40) arranged into the downstream conduct element (100).

15. Manual artificial respiration bag according to any one of the preceding claims, **characterized in that** it further comprises :
- a flexible gas reservoir (80) fluidly connected to the reservoir port (201) of the upstream conduct element (200), and/or
- a respiratory interface (70) fluidly connected the interface port (140) of the downstream conduct element (100), preferably by means of a ball-head connector (150), and/or
- an oxygen source fluidly connected (90) to the oxygen port (230) of the upstream conduct element (200).

## Amended claims

### Amended claims in accordance with Rule 137(2) EPC.

1. Manual artificial respiration bag (1) comprising:
- a deformable bag (2) comprising a gas inlet (4), a gas outlet (3) and an inner volume (5) for a respiratory gas,
- a downstream conduct element (100) fluidly connected to the gas outlet (3) of the deformable bag (2), and comprising an exhaust valve (110),
- a downstream one-way valve (50, 55) arranged into the downstream conduct element (100), said downstream one-way valve (50, 55) being configured for allowing a flow of respiratory gas to pass through said downstream one-way valve (50, 55) only toward the exhaust valve (110), and
- flow-resistance setting means (120) cooperating with the downstream one-way valve (50, 55) for setting or adjusting a desired flow resistance level of said downstream one-way valve (50, 55) thereby controlling the flow of respiratory gas passing through said downstream one-way valve (50, 55),
**characterized in that** :
- the downstream one-way valve (50, 55) comprises a valve support (55) arranged into the downstream conduct element (100) and a flexible valve body (50), and
- the flow-resistance setting means (120) comprise a mobile adjusting member (121), rotatable by a user, comprising a bearing surface (122) acting on the flexible valve body (50) of the downstream one-way valve (50, 55) for setting or adjusting the flow resistance level of said downstream one-way valve (50, 55).

2. Manual artificial respiration bag according to the preceding claim, **characterized in that** the flexible valve body (50) is sandwiched between the mobile adjusting member (121) and the valve support (55).

3. Manual artificial respiration bag according to any one of the preceding claims, **characterized in that** the flexible valve body (50) of the downstream one-way valve (50, 55) has an umbrella-shape.

4. Manual artificial respiration bag according to any one of the preceding claims, **characterized in that** :
- the flexible valve body (50) of the downstream one-way valve (50, 55) has an umbrella-shape comprising a disk-shape body (52) and a rod element (51) integral with said disk-shape body (52), and
- the valve-support (55) comprises a support orifice (56), the rod element (51) of the flexible valve body (50) traversing said support orifice (56) of the valve-support (55).

5. Manual artificial respiration bag according to any one of the preceding claims, **characterized in that** the bearing surface (122) of the mobile adjusting member (121) acts on the disk-shape body (52) of the flexible valve body (50) of the downstream one-way valve (50, 55), when actuated by the user, thereby setting or adjusting the flow resistance level of said downstream one-way valve (50, 55).

6. Manual artificial respiration bag according to any one of the preceding claims, **characterized in that** the adjusting member (121) comprises at least one guiding-pin (123) and the downstream conduct element (100) comprises at least one guiding-groove (101), said guiding-pin (123) being guided by and in said guiding-groove (101) when the adjusting member (121) is rotated by the user.

7. Manual artificial respiration bag according to claim 6, **characterized in that** the adjusting member (121) comprises two guiding-pins (123) and the downstream conduct element (100) comprises two guiding-groove (101).

8. Manual artificial respiration bag according to any one of the preceding claims, **characterized in that** it further comprises mobile port-closing means (124), actuatable by the user, cooperating with the exhaust port (111) of the exhaust valve (110) for at least partially closing said exhaust port (111), thereby controlling the flow of respiratory gas passing through the exhaust port (111) of the exhaust valve (110).

9. Manual artificial respiration bag according to claim 8, **characterized in that** the mobile port-closing means (124) are arranged on the adjusting member (121) so that said mobile port-closing means (124) at least partially close said exhaust port (111) in response to an actuation of the adjusting member (121) by the user, typically to a rotation of the adjusting member (121) by the user.

10. Manual artificial respiration bag according to any one of the preceding claims, **characterized in that** it further comprises an upstream conduct element (200) fluidly connected to the gas inlet (4) of the deformable bag (2), said upstream conduct element (200) comprising :
- a PEP exhaust valve (210) fluidly communicating with the ambient atmosphere for venting gas to the atmosphere when the gas pressure, into the upstream conduct element (200), exceeds a given pressure threshold, and
- an air admission valve (220) in fluid communication with the ambient atmosphere,
- and/or an oxygen port (230) for connecting an oxygen source.

11. Manual artificial respiration bag according to claim 10, **characterized in that** the PEP exhaust valve (210) arranged in the upstream conduct element (200) comprises PEP-setting means for setting the desired pressure threshold.

12. Manual artificial respiration bag according to any one of the preceding claims, **characterized in that** :
- the upstream conduct element (200) further comprises a reservoir port (201) for fluidly connecting a flexible gas reservoir (80), and
- the downstream conduct element (100) further comprises an interface port (140) for fluidly connecting a respiratory interface (70).

13. Manual artificial respiration bag according to any one of the preceding claims, **characterized in that** it further comprises :
- an upstream one-way valve (30) arranged into the upstream conduct element (200) between the deformable bag (2) and the PEP exhaust valve (210), said upstream one-way valve (30) being configured for allowing a flow of respiratory gas to pass through said upstream one-way valve (30) only toward the deformable bag (2), and/or
- a flow-restriction element (40) arranged into the downstream conduct element (100).

14. Manual artificial respiration bag according to any one of the preceding claims, **characterized in that** it further comprises :
- a flexible gas reservoir (80) fluidly connected to the reservoir port (201) of the upstream conduct element (200), and/or
- a respiratory interface (70) fluidly connected the interface port (140) of the downstream conduct element (100), preferably by means of a ball-head connector (150), and/or
- an oxygen source fluidly connected (90) to the oxygen port (230) of the upstream conduct element (200).
